# EUROPEAN PATENT APPLICATION

(11) **EP 2 184 061 A1**
(43) Date of publication of application: **12.05.2010**
(21) Application number: 08168691.7
(22) Date of filing: 07.11.2008
(51) Int. Cl.: A61K 31/045, A61K 31/11, A61K 31/231, A61P 31/02, A61P 31/04

(54) **Genariol as bacterial efflux pump inhibitor**

(71) Applicant: Universite de Corse, 20250 Corte (FR)
(72) Inventor: Berti, Liliane, 20200, BASTIA (FR); Lorenzi, Vannina, 20250, CORTE (FR); Casanova, Joseph, 20167, ALATA (FR); Muselli, Alain, 20222, ERBALUNGA (FR); Pages, Jean-Marie, 13005, MARSEILLE (FR); Bolla, Jean-Michel, 13009, MARSEILLE (FR)
(74) Representative: Chajmowicz, Marion

(57) **Abstract**

The present invention relates to the use of geraniol or any of its saturated or unsaturated monoterpene derivatives as a bacterial efflux pump inhibitor, for reducing antibiotic or antiseptic resistance or restoring antibiotic or antiseptic sensitivity to an antibiotic or antiseptic-resistant bacterial strain.

## Description

The constant use of antibiotics in the hospital environment has selected bacterial populations that are resistant to many antibiotics. In particular, *Enterobacter aerogenes,* a commensal Gram-negative bacterium of human intestinal flora, has been rapidly emerging as an important nosocomial pathogen (4, 8, 18, 19) with an increasing frequency of isolates resistant to many antibiotics and antiseptics (9). This multidrug resistance (MDR) can result from the down-regulation of non-specific porins which reduces the permeability of the cell envelope to antibiotics (13) as well as from increased active efflux of antibiotics prior to reaching their effective cytoplasmic targets (36).

The major efflux mechanism of *E. aerogenes* is the AcrAB-TolC tripartite pump (49). This pump belongs to the resistance-nodulation-division family (RND), spans the inner and outer membranes of the cell envelope and actively extrudes unrelated antibiotics from the periplasm to the environment. In addition, other less characterized efflux mechanisms contribute to the MDR phenotype of *E. aerogenes* clinical isolates (16, 49). Among the major strategies to overcome MDR bacterial resistance is the use of agents that inhibit an MDR efflux pump and therefore render the bacterium susceptible to antibiotics to which it was initially resistant (32, 34).

There are two approaches to tackling the problem of MDR resistance in bacteria. The identification of new targets and further molecules that selectively act on the targets to inhibit the growth of specific organisms (14, 47), and the identification of agents that have the capability to restore antibiotic susceptibility (46). The latter approach has historically involved massive screening of products generated by micro-organisms, modification of such products by either micro-organisms or organic chemistry procedures, and less frequently, actual *de novo* syntheses. Although to date no EPI has yet been made available for the therapy of MDR bacterial infections, some are in pre-clinical development (46). Plants have recently been seen as viable sources for antimicrobial agents (5, 24, 26, 30, 43, 51, 52, 55), but only a few studies have evaluated plants for compounds that have an affect on the susceptibility of MDR bacteria to antibiotics (1, 3, 12, 20, 38, 45).

Plant extracts have been shown to enhance the activity of antibiotics *in vitro* (40, 52) and *ex vivo* (38, 45) by their ability to inhibit efflux pumps. Methanol or ethanol extracts have been shown to inhibit efflux of ciprofloxacin and norfloxacin in Gram-negative bacteria (41) and *S. aureus* (10, 20), respectively. Hence, plants may provide potential sources of compounds which if non-toxic, may be used as adjuncts for rendering MDR bacterial infections susceptible to antibiotics (54). They may also serve as lead compounds for the development of new EPIs.

Essential oils have been shown to have antimicrobial activity against a wide range of bacteria (5, 11, 24, 26, 30, 43, 48, 53, 55) and some have been identified to contain EPIs (reviewed by 54). The island of Corsica has a great number of aromatic species that are likely to provide essential oils. Some of these essential oils have been widely studied (6, 7, 23, 33, 37, 50) and some have recently been shown to have antimicrobial activity (51).

The vast majority of EPIs are active against Gram-positive bacteria, particularly *Staphylococcus aureus.* The very few EPIs that are active against Gram negative bacteria are toxic, and Gram-negative bacteria such as *Pseudomonas, Acinetobacter, Escherichia,* and *Enterobacter* are rapidly becoming the most problematic bacteria to treat due to the expression of MDR phenotypes and nosocomial status.

There is thus a great need for EPIs that are effective in rendering MDR Gram negative bacterial infections susceptible to antibiotics to which they are initially resistant (15, 34, 35).

The inventors have now found out that essential oils obtained from Corsican plants contain compounds that restore antibiotic activities on multi-drug resistant bacteria, especially isolates from Gram-negative bacterial species.

More particularly the inventors have shown that fractions isolated from the essential oil of *Helichrysum italicum* (HI) significantly reduce MDR of *Enterobacter aerogenes, Escherichia coli, Pseudomonas aeruginosa* and *Acinetobacter baumannii.* Combinations of the two most active fractions with each other or with the EPI phenylalanine arginine β-naphthylamide yield synergistic EPI activity suggesting differential activity against distinct bacterial efflux pumps. Geraniol was identified as an active compound of the essential oil and significantly increased the efficacy of various antibiotic classes, including β-Lactams and quinolones in addition to chloramphenicol against the above panel of bacteria.

On this basis, geraniol or any of its derivatives is used as an inhibitor of bacterial efflux pumps. Insofar as antibiotics and antiseptics involve efflux pumps, the invention encompasses fighting against bacterial strains which have developed or are likely to develop antibiotic or antiseptic resistance.

More particularly, the present invention provides geraniol or its saturated or unsaturated monoterpene derivatives, for use in reducing antibiotic or antiseptic resistance or restoring antibiotic or antiseptic sensitivity to an antibiotic or antiseptic-resistant bacterial strain.

The invention founds application in medical, veterinary and non-medical, such as food industry, fields.

In a preferred embodiment, geraniol or any of its derivatives is for use in treating a subject infected with an antibiotic, or an antiseptic-resistant bacterial strain. The subject may be any animal likely to be infected with such strains, preferably a human being or a mammal, including cattle, sheep, horses, dogs, cats, goats etc. Poultry, fish or any other animals for food industry are also encompassed. Preferably the subject is a human patient, whatever its age or sexe. New-borns, infants, children are included as well.

Accordingly, it is herein disclosed a method for reducing antibiotic or antiseptic resistance or restoring antibiotic or antiseptic sensitivity to an antibiotic or antiseptic-resistant bacterial strain in a subject infected with such strain, which method comprises administering to said subject an effective amount of geraniol or any of its derivatives.

In a particular embodiment, geraniol or any of its derivatives is intended for use in local treatment of wounds infected with antiseptic or antibiotic resistant bacterial strains.

In another embodiment, geraniol or any of its derivatives is for use in disinfecting a product (i.e. a non-living product) infected or likely to be infected, with an antibiotic- or an antiseptic-resistant bacterial strain. In a particular embodiment, the product may also be a medical device. In another particular embodiment, the product may be a food product, e.g. a dairy or meat product, or a drink.

Accordingly, it is herein disclosed a method for reducing antibiotic or antiseptic resistance or restoring antibiotic or antiseptic sensitivity to an antibiotic or antiseptic - resistant bacterial strain in a product infected or likely to be infected with such strain, which method comprises applying to said product an effective amount of geraniol or any of its derivatives.

Geraniol is a terpene compound. Terpenes are derived biosynthetically from units of isoprene, which has the molecular formula C5H8:

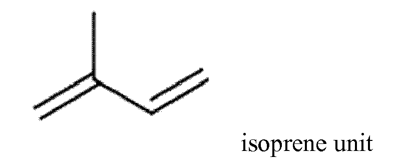

The basic molecular formulae of terpenes are multiples of that, (C5H8)n where n is the number of linked isoprene units. This is called the isoprene rule or the C5 rule. The isoprene units may be linked together "head to tail" to form linear chains or they may be arranged to form rings. One can consider the isoprene unit as one of nature's common building blocks. As chains of isoprene units are built up, the resulting terpenes are classified sequentially by size as hemiterpenes, monoterpenes, sesquiterpenes, diterpenes, sesterterpenes, triterpenes, and tetraterpenes. Terpenes may be classified by the number of terpene units in the molecule; a prefix in the name indicates the number of terpene units needed to assemble the molecule. A single terpene unit is formed from two molecules of isoprene, so that a monoterpene consists of one terpene but two isoprene units. Monoterpenes thus have the molecular formula C10H16.

Examples of genariol saturated or unsaturated monoterpene derivatives include nerol, geranyl acetate, neryl acetate, geranial, neral, citronellol, linalool, tetrahydrogeraniol, myrcenol, and geranyl acetol.

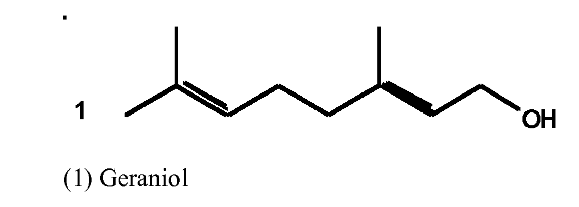

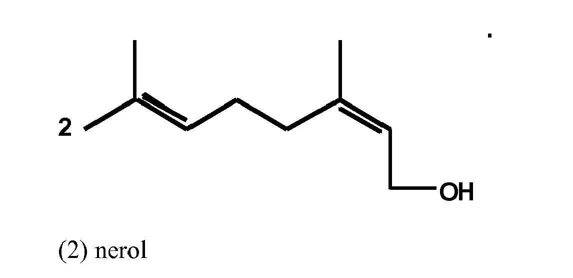

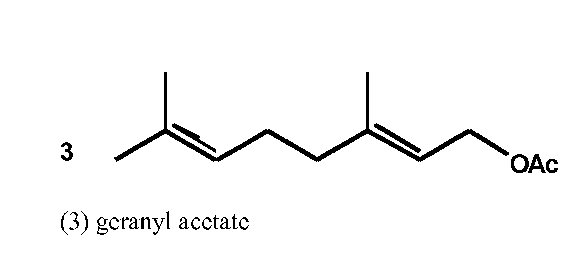

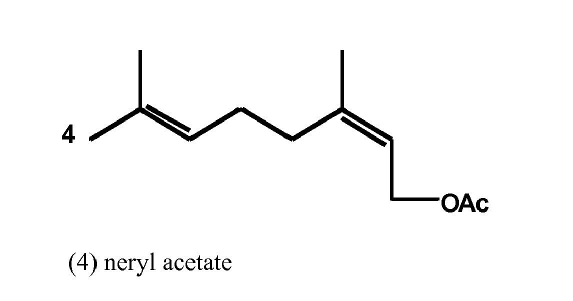

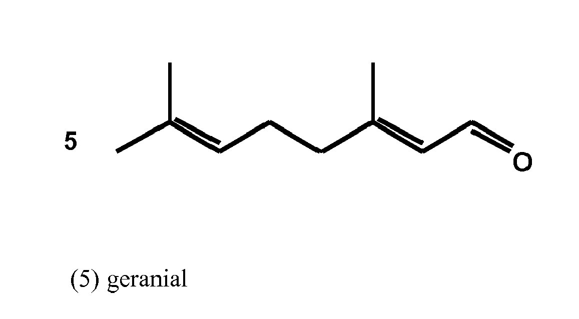

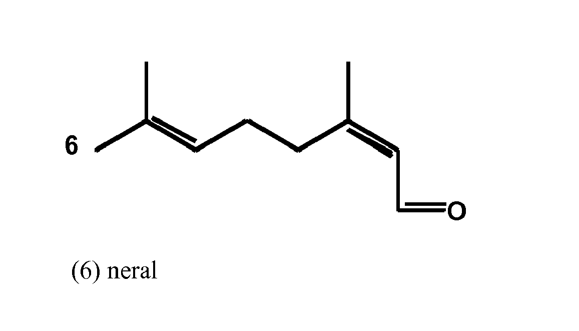

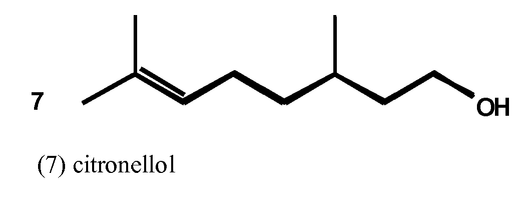

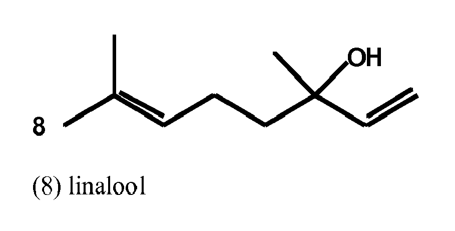

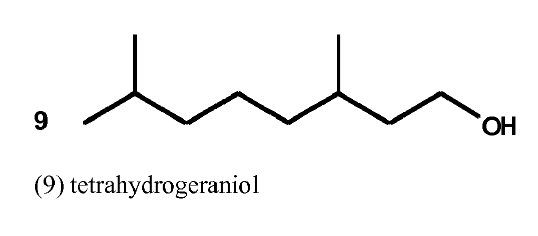

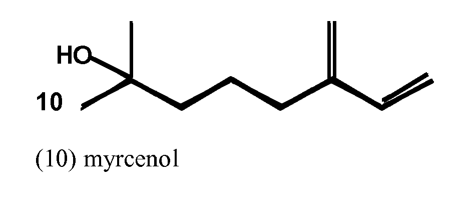

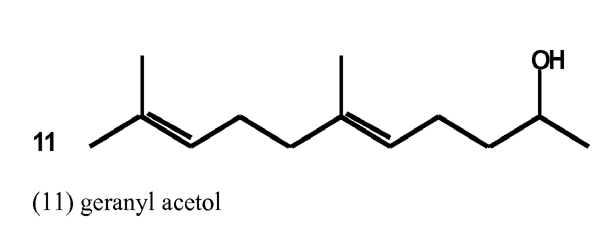

According to the present invention, geraniol or its derivatives is useful in reducing resistance or restoring sensitivity to any antibiotic or antiseptic-resistant bacteria, including Gram-negative bacteria or Gram-positive bacteria.

In a preferred embodiment, the bacterial strain is a Gram-negative bacterial strain.

The proteobacteria are a major group of Gram-negative bacteria, including *Escherichia coli, Salmonella,* and other *Enterobacteriaceae, Pseudomonas, Moraxella, Helicobacter, Stenotrophomonas, Bdellovibrio,* acetic acid bacteria, *Legionella* and alpha-proteobacteria as *Wolbachia* and many others. Other notable groups of Gram-negative bacteria include the cyanobacteria, spirochaetes, green sulfur and green non-sulfur bacteria.

Medically relevant Gram-negative cocci include three organisms, which cause a sexually transmitted disease (*Neisseria gonorrhoeae*), a meningitis (*Neisseria meningitidis*), and respiratory symptoms (*Moraxella catarrhalis*).

Medically relevant Gram-negative bacilli include a multitude of species. Some of them primarily cause respiratory problems (*Hemophilus influenzae, Klebsiella pneumoniae, Legionella pneumophila, Pseudomonas aeruginosa*), primarily urinary problems (*Escherichia coli, Proteus mirabilis, Enterobacter cloacae, Serratia marcescens*), and primarily gastrointestinal problems (*Campylobacter, Helicobacter pylori, Salmonella enteritidis, Salmonella typhi*).

Gram negative bacteria associated with nosocomial infections include *Acinetobacter baumanii,* which cause bacteremia, secondary meningitis, and ventilator-associated pneumonia in intensive care units of hospital establishments.

Geraniol or any of its derivatives is particularly useful in reducing antibiotic resistance or restoring antibiotic sensitivity in any of the above-mentioned Gram-negative bacteria.

In a preferred embodiment, geraniol or any of its derivatives is intended for use in a subject infected with enteropathogenic or uropathogenic bacterial strains. Preferably the patient is infected with *Enterobacter aerogenes, Escherichia coli, Pseudomonas aeruginosa* and *Acinetobacter baumannii,* or *Klebsellia, Salmonella, Campylobacter* or *Helicobacter* species.

Preferably the patient is infected with MDR strains of the above bacteria.

The mechanism behind MDR of pathogenic Gram negative bacteria is now accepted to be due to over-expressed efflux pumps that extrude a large number of unrelated antibiotics and antiseptics prior to reaching their intended targets. Gram negative bacteria contain a large number of efflux pumps which, at face value, appear, to be redundant (29).

Geraniol or its derivatives are preferably applied or administered in combination with an antibiotic or an antiseptic, the effect of which is increased by the action of geraniol or its derivative.

The term "combination" means that geraniol or any of its derivatives is applied or administered simultaneously or sequentially with the antibiotic or antiseptic. In the context of a medical treatment, geraniol or any of its derivatives, and the antibiotic or antiseptic are administered preferably the same day, most preferably within an interval of 2 hours, preferably 1 hour, at most.

Examples of antibiotics include beta-lactam antibiotics, macrolides (such as erythromycin, clarithromycin, and azitromycin), monobactams, rifamycins, tetracyclines, chloramphenicol, clindamycin, lincomycin, fusidic acid, novobiocin, fosfomycin, fusidate sodium, capreomycin, colistimethate, gramicidin, minocycline, doxycycline, bacitracin, quinolones (such as nalidixic acid), fluoroquinolones (such as ciprofloxacin), vancomycin, and trimethoprim.

In the context of the present invention, the term "antiseptics" refers to antibacterial agents, including bacteriocidal or bacteriostatic agents. Antiseptics include ethanol, isopropanol, glutaraldehyde, tricocarban, chlorhexidine, alexidine, polymeric biguanides, triclosan, hexachlorophene, propamidine, dibromopropamidine, chloroxylenol, chlorine or iodine releasing compounds, silver or mercury compounds, hydrogen peroxide, ozone, peracetic acid, phenol, cresol, cetrimide, benzalkonium chloride, ethylene oxide, formaldehyde, etc.

In the context of a medical or veterinary treatment, geraniol or any of its derivatives may be administered to a subject by any suitable route, including oral, topical, sublingual, parenteral (preferably intravenous), transdermal, rectal, etc.

For a brief review of present methods for drug delivery, see, Langer, Science 249:1527-1533 (1990), which is incorporated herein by reference. Methods for preparing administrable compounds are known or are apparent to those skilled in the art and are described in more detail in, for example, Remington's Pharmaceutical Science, 17th Ed., Mack Publishing Company, Easton, Pa. (1985), which is incorporated herein by reference, and which is hereinafter referred to as "Remington."

Topical administration on wounds may be particularly advantageous against Pseudomonas infections, for instance. Otherwise, oral administration may be particularly convenient.

In the veterinary field, topical administration, e.g. treatment of wounds, may be advantageous too.

In agricultural and food industry, a treatment of animals in slaughterhouse may be contemplated, e.g. by spraying or dipping the animal, *e*.*g*. poultry, or parts thereof, in washing baths.

When used for disinfecting a product, geraniol or any of its derivatives may be e.g. in form of a solution applied to the surface of the product, or a bath into which the product is dipped.

The below Examples illustrate the invention without reducing its scope.

### EXAMPLES:

### MATERIALS AND METHODS

### Bacterial strains, growth conditions, susceptibility tests and determination of effects of EPIs, essential oil and isolated compounds from essential oil on antibiotic susceptibility.

The bacterial strains used in this study are listed in Table 1.

**TABLE 1. Bacterial strains**

| Bacterial strains | Relevant features^{a} | CHL-MIC^{b} | Source or reference |
|---|---|---|---|
| *E. aerogenes* strains | | | |
| ATCC 13048 | Susceptible strain | 8 | ATCC |
| EA3 | MDR clinical isolate; | 512 | 36 |
| EA5 | MDR clinical isolate; | 512 | 36 |
| EA27 | MDR clinical isolate; | | |
| | | 1024 | 36 |
| | Kan^{r} Amp^{r} Chl^{r} Nal^{r} Str^{r} Tet^{r} | | |
| EAEP289 | Kan^{s} derivative of EA27 | 1024 | 49 |
| EAEP294 | EA289 *acrA*::Kan^{r} | 64 | 49 |
| CM-64 | derivative of ATCC 13048 | 512 | 22 |

| *E. coli* strains | | | |
|---|---|---|---|
| AG100 | Wild type *E. coli* K12 | 8 | 44 |
| AG100A | AG100 Δ*acrAB*::Kan^{r} | 0.5 | 44 |
| AG100A Tet^{r} | AG100A; Tet^{r} | 64 | 57 |

| *A. baumanii* strains | | | |
|---|---|---|---|
| ATCC 19606 | | 32 | 21 |
| AB1 | | 32 | 21 |

| *P. aeruginosa* strains | | | |
|---|---|---|---|
| PAO1 | Reference strain | 512 | 25 |
| PA124 | MDR clinical isolate | 128 | Laboratory collection |

| | | | |
|---|---|---|---|
| ^{a}Amp^{r}, Chl^{r}, Kan^{r}, Nal^{r}, Str^{r}, and Tet^{r}, resistance to ampicillin, chloramphenicol, kanamycin, nalidixic acid, streptomycin, and tetracycline, respectively. ^{b}CHL-MIC are given in mg/L. | | | |

The wild type *E. aerogenes* ATCC 13048, *E. coli* AG100 and *Acinetobacter baumanii* ATCC 19606 served as controls. EAEP289 strain is a Kan^{s} derivative of the MDR clinical isolate EA27. The *acrA* mutant, EAEP294, was constructed from EA289 (49). The chloramphenicol resistant EA CM-64 is a derivative from *E. aerogenes* ATCC 13048-type strain and exhibits significant resistance to chloramphenicol by over expression of the AcrAB-TolC pump complex (22). The *Escherichia coli* kanamycin resistant *acrAB* mutant, AG100A is hyper-susceptible to chloramphenicol, tetracycline, ampicillin and nalidixic acid (44). Tetracycline resistance induced in AG100A (AG100A Tet^{r}) results in the increased expression of all the efflux transporter genes, especially the *acrEF* genes (57). *A. baumanii* ATCC 19606-type strain and a clinical MDR isolates were previously described (21). The *Pseudomonas aeruginosa* strains PAO1 and the clinical MDR isolate PA124 were obtained from laboratory collection. Bacteria were routinely grown in Mueller-Hinton (MH) broth at 37°C.

For the determination of minimum inhibitory concentrations (MICs%) 10⁶ cells were inoculated in 1 ml of MH broth containing two-fold serial dilutions of each essential oil. The cultures were incubated at 37C and visually examined 18 h later (13, 36). The MIC % represented the lowest concentration (% v/v) of essential oil where absence of growth was recorded.

The increased susceptibility to given antibiotics produced by the presence of an EPI essential oil or chemical compounds (antibiotic restoration test) were performed by the same dilution method described above. Various concentrations of antibiotics, alone or in the presence of phenylalanine arginine β-naphthylamide (PAβN) or essential oil were tested. PAβN was used at 20 mg/L as described by Lomovskaya *et al.* (31). The amount of essential oil was equivalent to less than 1/4 of its MIC (if any) in order to assure that any reduction of antibiotic resistance would be due to the EPI activity of the oil and not due to a direct inhibition of bacterial growth (56). For each sample resulting from chromatographic fractionation of *H. italicum* essential oil, an MIC determination was determined before its use in the antibiotic restoration tests. The amount of isolated fraction employed for the latter tests was equivalent to ¼ its MIC%. All MIC and antibiotic restoration tests were repeated three times and the results did not exceed +/- 1 dilution from the average value provided in the tables and text.

**Essential oils and chemicals.** The essential oils were either purchased at "Huiles Essentielles et Hydrolats de Corse" (Mandriolu, Corsica, France) or obtained from the dried aerial parts of H. italicum by hydrodistillation for 4 h using a Clevenger-type apparatus in the laboratory (17). The isolated essential oils were filtered on millipore nitrocellulose filters (0.22 µm) and stored at 4°C until their used in bioassays. Neryl acetate and geraniol were purchased from Sigma-Aldrich (St Quentin-Fallavier, France).

**Fractionation of *H. italicum* essential oil.** The *H. italicum* essential oil (8 g) was chromatographed on a silica gel column (ICN 200-500 µm, 150 g) and serially eluted with pentane (n-C₅H₁₂) and diethyl ether (ET₂O) for separation of the hydrocarbons compounds (FH: 1.3 g) and oxygenated compounds (FO: 6 g), respectively. A part of the oxygenated fraction (4.5 g) was further fractionated on silica gel (70-230 µm, 250 g) with a solvent gradients of n- C5H12:ET₂O of 98:2, 90:10, 0:100 to yield three fractions: F1 (2.5 g), F2 (850 mg) and F3 (900 mg).*!*

**Analysis conditions.** *GC analysis.* GC analyses were carried out using a Perkin Elmer Autosystem GC apparatus equipped with a single injector and two flame ionization detectors (FID);the apparatus was used for simultaneous sampling to two fused-silica capillary columns (60 m x 0.22 mm i.d., film thickness 0.25 µm) with different stationary phases:Rtx-1 (polydimethylsiloxane) and Rtx-Wax (polyethylene glycol). Oven temperature programmed from 60°C to 230°C at 2°C/min and then held isothermal (30 min). Carrier gas: helium (1ml/min). Injector and detector temperatures were held at 280°C. Split injection was conducted with a ratio split of 1:80. Injected volume: 0.1 µL. The relative percentage of the oil constituents was calculated from the GC peaks areas without using correction factors.

*GC-MS analysis.* The oils and all fractions were analyzed with a Perkin Elmer TurboMass detector, directly coupled to a Perkin Elmer Autosystem XL equipped with two fused-silica capillary columns (60 m x 0.22 mm i.d., film thickness 0.25 µm), Rtx-1 (polydimethylsiloxane) and Rtx-Wax (polyethylene glycol). Other GC conditions were the same as described under GC. Ion source temperature: 150°C; energy ionization: 70 eV; electron ionization mass spectra were acquired over the mass range 35-350 Da. Oil injected volume: 0.1 µL, fractions injected volume: 0.2 µL.

*Component identification.* Identification of the components was carried out by comparison of their mass spectra with those compiled in 4 commercial mass spectra libraries (2, 28, 39, 42) and with those compiled in laboratory-made spectral library, as well as by comparison of their retention indices with those of authentic samples or literature data (27, 28). The GC retention indices (RI) on polar and non polar columns were determined relative to the retention time of a series of n-alkanes with linear interpolation.

### RESULTS

**Susceptibility of *Enterobacter* MDR strains to Corsican essential oils.** The susceptibility of the clinical isolate EA27 which over-expresses the acrAB complex due to a frameshift mutation in the regulator *acrR* (49) to essential oils derived from Corsican plants is summarized by Table 2.

**TABLE 2. MIC (% v/v) of the essential oils against the MDR EA27 clinical strain and their effects on the susceptibility of the strain to chloramphenicol**

| Substance | MIC (% v/v) | CHL-MIC (mg/L) | Fold restoration |
|---|---|---|---|
| No | - | 1024 | |
| *Cistus ladaniferus* | 2.5% | 512 | 2 |
| *Citrus aurantium* | >5% | 256 | 4 |
| *Citrus sinensis* | *>5%* | 512 | 2 |
| *Crythmum maritimum* | >5% | 1024 | 1 |
| *Cupressus sempervirens* | >5% | 1024 | 1 |
| *Daucus carota* | *>5%* | 512 | 2 |
| *Foeniculum vulgare* | >5% | 512 | 2 |
| *Helichrysum italicum* | >5% | 128 | 8 |
| *Inula graveolens* | >5% | 256 | 4 |
| *Lavandula stoechas* | 1.25% | 256 | 4 |
| *Mentha acquatica* | >5% | 512 | 2 |
| *Myrtus communis* | >5% | 512 | 2 |
| *Pinus lariccio* | 5% | 512 | 2 |
| *Pistacia lentiscus* | >5% | 512 | 2 |
| *Santolina corsica* | 0.625% | 1024 | 1 |
| PAβN | - | 64 | 16 |

The range of antimicrobial activity of these Corsican essential oils varied from a low of > 5% (v/v) and to highs of 1.25 and 0.626%, the latter MICs for the oils derived from *Lavandula stoechas* and *Santolina corsica,* respectively. The effect of these essential oils at 1/4 their MIC on the susceptibility of EA27 to chloramphenicol is also presented by Table 2. The effect of PAβN on chloramphenicol resistance is also presented in order that the EPI effect of each essential oil (EPIA) can be appreciated. Briefly, whereas the essential oil derived from *Helichrysum italicum* reduces the MIC of chloramphenicol from 1024 to 128 mg/L (8 fold); the remainder are less active and reduce the MIC of chloramphenicol from 2 to 4 folds. Due to the ability of the essential oil derived from *H. italicum* to reduce chloramphenicol resistance of EA27 as well as to two other MDR strains EA3 and EA5 (data not shown) to a level that is close to that of the control PAβN, it was selected for further study that would evaluate its ability to reduce antibiotic resistance of other Gram negative bacteria.

***H. italicum* essential oil modulates the susceptibility of Gram-negative species to chloramphenicol.** *A. baumannii* and *P. aeruginosa* are now among the most problematic bacteria to treat (54). Compounds that can enhance the activity of antibiotics to which these species have become increasingly resistant may be essential for the control of these pathogens. As demonstrated by Table 3, the essential oil derived from *H. italicum* is more effective than PAβN in reducing the resistance of *A. baumannii* strains to chloramphenicol.

**TABLE 3. Effect of H. italicum essential oil on the susceptibility of Gram negative species to chloramphenicol**

| | Chloramphenicol MIC (mg/L) (fold restoration) | | | |
|---|---|---|---|---|
| | *A. baumanii* | | *P. aeruginosa* | |
| Substance | ATCC 19606 | AB1 | PAO1 | PA124 |
| No | 32 | 32 | 512 | 128 |
| PAβN (20 mg/L) | 16(2) | 16(2) | 2(256) | 4(32) |
| *H. italicum* essential oil | 4(8) | 4(8) | 32(16) | 16(8) |

In the case of the highly chloramphenicol resistant *P. aeruginosa* strains, although it reduces resistance to a greater extent than that produced in *A. baumannii,* it is not as effective as PAβN. The ability of the *H. italicum* essential oil to significantly reduce chloramphenicol resistance of three very pathogenic MDR Gram negative bacteria supports the prediction of similar activity against other Gram negative pathogens.

**Activity of *H. italicum* essential oil on *E. aerogenes* and *E. coli* efflux pump-mutants.** Evaluation of potential EPIs is best conducted with bacteria that over-express a specific efflux pump that renders the bacterium MDR. The ability of the *H. italicum* essential oil to reduce chloramphenicol resistance of the EA27, a strain that over-expresses its acrAB efflux pump that renders the organism resistant to antibiotic, suggests that this essential oil contains an agent with the activity of an EPI. That this suggestion is correct is evident from the data summarized by Table 4.

**TABLE 4. Effect of H. italicum essential oil on the susceptibility of E. aerogenes and E. coli efflux-mutant strains to chloramphenicol**

| | Chloramphenicol MIC (mg/L) (fold restoration) | | | | | | |
|---|---|---|---|---|---|---|---|
| Substance | EAEP289 | EAEP294 | ATCC13048 | EA CM-64 | AG100 | AG100A | AG100A Tet^{r} |
| No | 1024 | 64 | 8 | 512 | 8 | 0.5 | 64 |
| PAβN | 64(16) | 32(2) | 2(4) | 16(32) | 2(4) | 0.5(1) | 2(32) |
| HI(*) | 128(8) | 0.5(128) | 4(2) | 64(8) | 2(4) | 1(0.5) | 0.25(256) |
| HI(*) + PAβN | 4(256) | - | - | - | - | - | - |

| | | | | | | | |
|---|---|---|---|---|---|---|---|
| (*) *Helychrisum italicum* essential oil | | | | | | | |

The presence of the essential oil reduces chloramphenicol resistance not only of the MDR strain EAEP289 that over express efflux pumps but also intrinsic chloramphenicol resistance of the controls *E. aerogenes* ATCC13048 and AG100 wild types. Moreover, a strong restoration was observed in the presence of the essential oil on strain EA CM-64 that over produced acrAB (22) and also on the MDR strain EAEP294 that was deleted of the *acrAB* operon (49) and still has other active efflux pumps (16). In contrast to these effects, the *E. coli* mutant AG 100A, which has its *acrAB* operon deleted, is not affected by the essential oil. Because the resistance of the *acrAB* deleted progeny which has been induced to high-level resistance to chloramphenicol is due to the over-expression of the AcrEF system (57), the reduced resistance to chloramphenicol noted for the *acrAB* Tet^{R} mutant suggests that the essential oil is active against AcrEF. Regardless, from the data summarized by Table 4, it is clear that the essential oil derived from *H. italicum* contains one or more agents that have EPI activity and hence, an attempt to isolate that agent or agents was made.

**Fractionation of *H. italicum* essential oil.** The compounds identified in the *H. italicum* essential oil and its fractions are summarized in Table 5.

**TABLE 5. Chemical composition (%) of H. italicum essential oil and fractions obtained by repeated chromatography**

| | Sample | | | | | | |
|---|---|---|---|---|---|---|---|
| Components | RI^{a} | RI^{p} | HI | FO | F1 | F2 | F3 |
| 4-Methylhexan-3-one | 798 | 1059 | 0.3 | 0.3 | - | - | - |
| α-Pinene | 931 | 1007 | 2.5 | - | - | - | - |
| α-Fenchene | 942 | 1035 | 0.5 | - | - | - | - |
| Camphene | 944 | 1044 | 0.2 | - | - | - | - |
| β-Pinene | 971 | 1088 | 0.6 | - | - | - | - |
| Myrcene | 981 | 1140 | 0.4 | - | - | - | - |
| α-Phellandrene | 989 | 1204 | t | - | - | - | - |
| α-Terpinene | 1010 | 1255 | 0.1 | - | - | - | - |
| *p*-Cymene | 1013 | 1236 | 0.4 | - | - | - | - |
| Limonene | 1023 | 1173 | 6.0 | - | - | - | - |
| 1,8-Cineole | 1023 | 1189 | 1.1 | 0.7 | 0.6 | - | - |
| (E)-β-Ocimene | 1037 | 1218 | 0.2 | - | - | - | - |
| α-Terpinene | 1049 | 1215 | 0.5 | - | - | - | - |
| 3,5-Dimethylheptan-2,4-dione | 1069 | 1510 | 0.6 | 0.6 | - | 2.3 | - |
| Nonan-2-one | 1071 | 1362 | 0.4 | 0.4 | - | 1.6 | - |
| Terpinolene | 1080 | 1259 | 0.2 | - | - | - | - |
| Linalool | 1086 | 1516 | 2.6 | 2.9 | - | - | 14.7 |
| *Trans*-pinocarveol | 1130 | 1650 | t | t | 0.1 | - | - |
| Nerol oxide | 1137 | 1446 | 0.6 | 0.6 | 0.8 | - | - |
| 4,6-Dimethylheptan-3,5-dione | 1162 | 1580 | 2.2 | 2.7 | - | 14.1 | - |
| Terpin-1-en-4-ol | 1164 | 1580 | 0.4 | 0.4 | - | - | 2.5 |
| α-Terpineol | 1175 | 1663 | 0.8 | 0.9 | - | - | 5.4 |
| Nerol | 1213 | 1765 | 2.6 | 3.4 | - | 2.1 | 19.4 |
| Pulegone | 1215 | 1645 | 0.3 | 0.4 | - | - | - |
| Geraniol | 1237 | 1843 | t | 0.1 | - | - | 0.2 |
| Geranial | 1244 | 1679 | 0.1 | 0.1 | - | - | 0.1 |
| Neryl acetate | 1351 | 1699 | 34.2 | 45.3 | 77.8 | - | - |
| Geranyl acetate | 1366 | 1726 | 0.4 | 0.6 | 0.6 | - | - |
| Isoitalicene | 1379 | 1456 | 0.8 | - | - | - | - |
| Italicene | 1406 | 1502 | 2.8 | - | - | - | - |
| (E)-α-bergamotnen | 1412 | 1566 | 0.5 | - | - | - | - |
| 4,6,9-Trimethyldec-8-en-3,5-dione | 1420 | 1861 | 4.2 | 5.7 | - | 34.3 | - |
| Neryl propionate | 1436 | 1754 | 5.3 | 7.1 | 12.0 | - | - |
| (E)-β-Farnesene | 1448 | 1621 | 0.1 | - | - | - | - |
| α-humulene | 1463 | 1641 | 0.2 | - | - | - | - |
| 2,4,6,9-Tetramethyldec-8-en-3,5-dione (*) | 1467 | 1861 | 2.1 | 2.8 | - | 15.8 | - |
| 2,4,6,9-Tetramethyldec-8-en-3,5-dione (*) | 1469 | 1861 | 2.8 | 3.6 | - | 16.5 | - |
| α-Curcumene | 1476 | 1651 | 10.4 | - | - | - | - |
| Guaiol | 1590 | 2046 | 0.8 | 1.2 | - | - | 6.0 |
| Eudesm-5-en-11-ol | 1602 | 2078 | 1.8 | 2.6 | - | - | 15.1 |
| Guai-5-en-11-ol | 1626 | | 0.9 | 1.4 | - | - | 8.1 |
| β-Eudesmol | 1641 | 2183 | 0.5 | 0.8 | - | - | 4.9 |
| α-Eudesmol | 1646 | 2177 | 0.5 | 0.8 | - | - | 4.8 |
| Bulnesol | 1657 | 2164 | 0.3 | 0.4 | - | - | 2.3 |
| α-Bisabolol | 1670 | 2173 | t | 0.1 | - | - | 0.4 |
| Total | | | 92.2 | 85.9 | 91.9 | 86.7 | 83.9 |

| | | | | | | | |
|---|---|---|---|---|---|---|---|
| Order of elution and percentages of individual components are given on Rtx-1 column. RI^{a} and RI^{p}: retention index Rtx-1 apolar column and Rtx-Wax polar column, respectively. t: trace (<0.005). (*) GC-MS and ¹³C-NMR investigations showed that these compounds are diastereoisomers (6). Compounds that are underlined were assayed independently, see below. | | | | | | | |

The oxygenated fraction FO (about 80 % of the total essential oil) was isolated from the hydrocarbons (about 20 % of the total essential oil) by flash chromatography. Three fractions were further obtained after chromatography of the oxygenated fraction. The first fraction (F1), that eluted with a mixture of pentane and ethyl-ether (98:2) represented about 50 % of the total oxygenated fraction and was mainly constituted with acetates compounds. The major compound of this fraction was neryl acetate (77.8 %). The second fraction (F2), obtained with a mixture of pentane and ethyl-ether (90: 10), represented about 20 % of the total oxygenated fraction and was dominated by β-diketones. The major component of this fraction was 4,6,9-Trimethyldec-8-en-3,5-dione (34.3 %), followed by 5,7,10-Trimethyldec-9-en-4,6-dione (16.5 %), 2,4,6,9-Tetramethyldec-8-en-3,5-dione (15.8 %) and 4,6-Dimethylheptan-3,5-dione (14.1 %). The last fraction, obtained with polar solvent (F3), represented about 30 % of the total oxygenated fraction and consisted mainly of alcohols. The major compound of this fraction was nerol (19.4 %), followed by eudesm-5-en-11-ol (15.1 %) and linalool (14.8 %).
Evaluation of the main fractions of the essential oil derived from *H. italicum* for EPI activity against the MDR EAEP289 strain was conducted and Table 6 summarizes the results obtained.

**TABLE 6. The EPI activity of H. italicum fractions alone or in combination with each other or with the EPI PAβN against E. aerogenes strains**

| | Chloramphenicol MIC (mg/L) on *E. aerogenes* strains (fold restoration) | |
|---|---|---|
| Fractions | EAEP289 | EAEP294 |
| No | 1024 | 64 |
| F2 | 512(2) | 32(2) |
| F3 | 512(2) | 0.5(128) |
| F2 + F3 | 128(8) | < 0.25 (>256) |
| F2 + PAβN | 4(256) | 4(16) |
| F3 + PAβN | < 0.25(>4096) | < 0.25(>256) |
| Geraniol | 64(16) | <0,25(>256) |
| Geraniol + PAβN | <0,25(>4096) | <025(>256) |

Briefly, whereas the hydrocarbon and F1 fractions are devoid of any EPI activity (i.e. reduction of chloramphenicol resistance) when used alone against the MDR EAEP289 strain, fractions F2 and F3 produce two fold reductions of chloramphenicol resistance. However, when fractions F2 and F3 are combined, they reduce chloramphenicol resistance from an initial MIC of 1024 to 128 mg/L. Reduction of resistance can also be achieved with combinations of PAβN with either F2 or F3 fractions, with combinations of the latter producing the greatest reduction that is comparable to a complete reversal of chloramphenicol resistance expected of a chloramphenicol hyper-susceptible strain. (i.e. MIC of less than 0.25 mg/L). Identical evaluation of each fraction alone or in combination with each other or with PAβN for EPI activity against the *acrAB* derivative EAEP294 strain demonstrated the very strong activity of fraction F3.. The inventors thus decided to perform chloramphenicol susceptibility testing in the presence of various compounds of F3 that were available.

**Identification of geraniol as the active compound of F3 :** Amongst the compound identified in F3, geraniol produced significant restoration of susceptibility of the MDR EAEP289 to chloramphenicol by as musch as 16 fold. When combined with PAβN, it rendered the organism fully susceptible to chloramphenicol, i.e., it completely reversed initial resistance (Table 6). Interestingly, geraniol restored the susceptibility of the *acrAB* derivative EAEP294 to the same extent as did the combination of F3 and PAβN (Table 6). The other compounds identified in F3 (underlined in Table 5) did not significantly reduce the resistance to chloramphenicol (data not shown).

### Restoration of susceptibility to β-Lactams and quinolones :

Chloramphenicol was used in this study to reveal efflux inhibition and to identify, amongst the different molecules contained in the essential oil, the active compound. Clinical isolates of Gram negatives exhibit resistance to these antibiotics by target modifications and/or by efflux. Herein the inventors have assayed the efficacy of geraniol in restoring antibiotic susceptibility , or at least increase susceptibility of EA289 and EA294 to the beta lactams ampicillin and penicillin and to the fluoroquinolone norfloxacin . The results are summarized in table 7.

**TABLE 7. The EPI activity of Geraniol on β-Lactams and Quinolone susceptibility**

| Strain | Ampicillin | Ampicillin + Geraniol | Penicillin | Penicillin + Geraniol | Norfloxacin | Norfloxacin + Geraniol |
|---|---|---|---|---|---|---|
| EA 289 | >1024 | >1024 | >1024 | >1024 | 256 | 128 |
| EA 294 | 512 | <0.07 | > 1024 | <0.07 | 64 | <0.07 |

As shown, the MICs of these antibiotics are very high, especially for the two β-lactams. When geraniol was added we were not able to observe a change of susceptibility of strain EA289. However, for EA294, the acrAB deficient strain, a very strong increase of susceptibility was observed in the presence of geraniol and a beta lactam. This indicated that the geraniol had a synergistic effect with β-lactams. Moreover, the same results were obtained with the fluoroquinolone norfloxacin, thus demonstrating a pleiotropic effect of geraniol against MDR phenotype in *E. aerogenes.*

**EPI-activity ranking.** The activity of agents that reduce antibiotic resistance of bacteria due to an inhibition of one or more efflux pumps that are the cause of such a resistance has to date, never been defined. We have begun the ranking of agents that demonstrate EPI activity relative to the reduction of specific antibiotic resistance produced by the EPI PAβN against a given Gram-negative strain. The simple formula: EPIA = Fold reduction of antibiotic activity produced by the agent divided by that produced by PAβN yields an index of EPI activity of the agent for any given bacteria-antibiotic combination for which PAβN is effective. A demonstration of this EPIA ranking for the fractions obtained from the essential oil of *H. italicum* alone or in combination with PAβN on the reduction of resistance to chloramphenicol is presented by Table 8.

**TABLE 8. Ranking of EPI activity (EPIA) of essential oils, essential oil fractions and purchased agents against control and efflux-pump Gram negative mutants relative to the control EPI PAβN**

| | PAβN | HI(*)/PAβN | F2/PAβN | F3/PAβN | F2 + F3/PAβN | Geraniol/ PAβN |
|---|---|---|---|---|---|---|
| **EAEP 289 MDR** | 16 | 0.5 | 0.125 | 0.125 | 0.5 | 1 |
| **EAEP 294 (*acrAB*)** | 2 | 64 | 1 | 64 | >128 | >128 |

| | | | | | | |
|---|---|---|---|---|---|---|
| Determination of EPIA: Fold reduction of antibiotic resistance produced by EPI (essential oil or fraction or purchased agent) divided by the fold reduction produced by PAβN. (*) *Helichrysum italicum* essential oil. | | | | | | |

The data presented is limited to the MDR EAEP 289 and EAE P294 (*acrAB* mutant) strains for the purpose of illustrating how such ranking may be used for quantifying reduction of a given MIC by a given EPI. A more comprehensive study will be reported elsewhere. Briefly, the EPIA activity of PAβN for the MDR strain EAEP 289 when compared to that of EAEP 294 is far greater, respective EPIA values of 16 and 2, respectively. Conversely, the EPIA activity of the essential oil of HI relative to that of PAβN, is far lower for EAEP 289 as opposed to that for EAEP 294, with values of 0.5 and 64, respectively. These observations suggest that the ability of the essential oil to reduce the MIC of each strain to chloramphenicol is significantly greater with the *acrAB* mutant as opposed to the MDR strain. These findings suggest that the MDR strain over-expresses a PAβN sensitive efflux system that is not overtly affected by the HI essential oil, whereas for the *acrAB* mutant, the EPI activity relative to that produced by PAβN, is substantially greater. The response of each strain to the essential oil may indicate the existence of distinct mechanisms, which are involved in degree to which the untreated strain is resistant to chloramphenicol. The fractionation of the HI essential oil suggests that the active EPIA is localized in the F3 and not in the F2 and only with respect to the *acrAB* mutant. Combining F3 and F2 yield EPIA only with the *acrAB* mutant, and this activity is far greater than that presented by the F3 fraction alone. Although the results suggest that it is the *acrAB* mutant that is primarily made more susceptible to chloramphenicol by the essential oil and its fractions, combinations of F3 with PAβN increase susceptibility to chloramphenicol of both strains. These latter results suggest that the combination of F3 with PAβN produces synergism due to EPIA affecting at least two separate systems involved in the chloramphenicol transport by the strain, namely, one affected by PAβN and the other affected by F3. Geraniol that was identified as the most active compound of F3 in this study showed an EPIA activity comparable to that of PAβN for the MDR strain EAPE289 (geraniol/PAβN = 1). Interestingly, the EPIA activity of geraniol is far greater compared to that of PAβN (geraniol/PAβN > 128) which is in accordance with the results obtained with F3. These results demonstrate that geraniol is a very potent inhibitor of chloramphenicol resistance mechanism in a strain devoid of acrAB. While a synergistic effect of geraniol and PAβN might occur it was difficult to observe due to the strong effect obtained with geraniol alone at a concentration of MIC/4 (see the material and methods section). However, when the assay was performed with PAβN in combination to geraniol at MIC/8 we observed a synergistic effect (data not shown). Taking together, these results suggest that PAβN and geraniol have an inhibitory effect on different mechanisms that are altogether involved in resistance.

### DISCUSSION

In this study the inventors demonstrated that the essential oil from *Helichrysum italicum* (HI) contains compounds that modulate drug resistance in several Gram-negative bacterial species by targeting efflux mechanisms. This conclusion is based on the following evidence. First, HI decreases the chloramphenicol MIC of *E. aerogenes* isolates (Tables 2 and 4) in addition to *A. baumannii* and *P. aeruginosa* strains (Table 3). Second, HI decreases the MIC of chloramphenicol of a strain that over-produced the tripartite efflux pump AcrAB-TolC (the strain EA CM-64, Table 4), and in addition it is able to restore susceptibility in a strain that over-expresses several efflux pumps different from AcrAB, the strain AG100A Tet^{r} (Table 4). The two fractions that are the most active contain compounds that have not been previously described as modulators (Tables 5 and 6). Together, these findings provide a new source of drugs that are helpful in the fighting against multi-drug resistant Gram-negative bacteria that continue to pose a threat to public health.

### REFERENCES

1. Abulrob, A. N., M. T. E. Suller, M. Gumbleton, C. Simons, and A. D. Russell. 2004. Identification and biological evaluation of grapefruit oil components as potential novel efflux pump modulators in methicillin-resistant Staphylococcus aureus bacterial strains. Phytochem. 65:3021-3027.
2. Adams, R. P. 2001. Software library called Identification of Essential Oil Components by Gaz Chromatography/Quadrupole Mass Spectroscopy. Illinois: Allured Publishing.
3. Amaral, L., M. Viveiros, and J. E. Kristiansen. 2006. "Non-Antibiotics": alternative therapy for the management of MDRTB and MRSA in economically disadvantaged countries. Curr. Drug Targets. 7(7):887-891.
4. Arpin, C., C. Coze, A. M. Rogues, J. P. Gachie, C. Bebear, and C. Quentin. 1996. Epidemiological study of an outbreak due to multidrug-resistant Enterobacter aerogenes in a medical intensive care unit. J. Clin. Microbiol. 34:2163-2169.
5. Baratta, M. T., H. J. D. Dorman, S. G. Deans, A. C. Figueiredo, J. G. Barroso, and G. Ruberto. 1998. Antimicrobial and antioxidant properties of some commercial essential oils. Flavour Fragr. J. 13:235-244.
6. Bianchini, A., P. Tomi, J. Costa, and F. Bernardini. 2001. Composition of Helichrysum italicum (roth) G. Don fil. subsp. italicum essential oils from Corsica (France). Flavour and Fragr. J. 16:30-34.
7. Blanc, M. C., A. Muselli, P. Bradesi, and J. Casanova. 2004. Chemical composition and variability of the essential oil of Inula graveolens from Corsica. Flavour Fragr. J. 19:314-319.
8. Bornet, C., A. Davin-Regli, C. Bosi, J. M. Pagès, and C. Bollet. 2000. Imipenem resistance of Enterobacter aerogenes mediated by outer membrane permeability. J. Clin. Microbiol. 38:1048-1052.
9. Bosi, C. A., A. Davin-Regli, C. Bornet, M. Malléa, J. M. Pagès, and C. Bollet. 1999. Most Enterobacter aerogenes strains in France belong to prevalent clone. J. Clin. Microbiol. 37:2165-2169.
10. Braga, L. C., A. A. Leite, K. G. Xavier, J. A. Takahashi, M. P. Bemquerer, E. Chartone-Souza, and A. M. Nascimento. 2005. Synergic interaction between pomegranate extract and antibiotics against Staphylococcus aureus. Can. J. Microbiol. 51:541-547.
11. Cavanagh, H. M. A., and J. M. Wilkinson. 2002. Biological activities of lavender essential oil. Phytother. Res. 16:301-308.
12. Cégélia-Carlioz, P., J. M. Bessière, B. David, A. M. Mariotte, S. Gibbons, and M. G. Dijoux-Franca. 2005. Modulation of multi-drug resistance (MDR) in Staphylococcus aureus by Osha (Ligusticum porteri L., Apiaceae) essential oil compounds. Flavour Fragr. J. 20:671-675.
13. Charrel, R. N., J. M. Pagès, P. De Micco, and M. Malléa. 1996. Prevalence of outer membrane porin alteration in β-lactam-antibiotic-resistant Enterobacter aerogenes. Antimicrob. Agents Chemother. 40:2854-2858.
14. Chen, D., and Z. Yuan. 2005 Therapeutic potential of peptide deformylase inhibitors. Expert Opin. Investig. Drugs. 14(9):1107-16.
15. Chevalier, J., J. Bredin, A. Mahamoud, M. Mallea, J. Barbe, and J. M. Pagès. 2004. Inhibitors of antibiotic efflux in resistant Enterobacter aerogenes and Klebsiella pneumoniae strains. Antimicrob. Agents Chemother. 48:1043-1046.
16. Chollet, R., J. Chevalier, C. Bollet, J. M. Pagès, and A. Davin-Regli. 2004. RamA is an alternative activator of the multidrug resistance cascade in Enterobacter aerogenes. Antimicrob. Agents Chemother. 48:2518-2523.
17. Conseil de l'Europe, Pharmacopée Européenne, Maisonneuve S.A.: Sainte Ruffine, 1996.
18. Davin-Regli, A., D. Monnet, P. Saux, C. Bosi, R. Charrel, A. Barthelemy, and C. Bollet. 1996. Molecular epidemiology of Enterobacter aerogenes acquisition: one-year prospective study in two intensive care units. J. Clin. Microbiol. 34:1474-1480.
19. De Gheldre, Y., M. J. Struelens, Y. Glupczynski, P. De Mol, N. Maes, C. Nonhoff, H. Chetoui, C. Sion, O. Ronveaux, M. Vaneechoutte, and le Groupement pur le Dépistage, I'Etude et la Prevention des Infections Hospitalières (GDEPIH-GOSP1Z). 2001. National epidemiologic surveys of Enterobacter aerogenes in Belgian hospitals from 1996 to 1998. J. Clin. Microbiol. 39:889-896.
20. Dickson, R. A., P. J. Houghton, P. J. Hylands, and S. Gibbons. 2006. Antimicrobial, resistance-modifying effects, antioxidant and free radical scavenging activities of Mezoneuron benthamianum Baill., Securinega virosa Roxb. & Wlld. and Microglossa pyrifolia Lam. Phytother. Res. 20:41-45.
21. Dupont, M., J. M. Pagès, D. Lafitte, A. Siroy, and C. Bollet. 2005. Identification of an OprD homologue in Acinetobacter baumanii. J. Proteome Res. 4(6):2386-2390.
22. Ghisalberti, D., M. Masi, J. M. Pagès, and J. Chevalier. 2005. Chloramphenicol and expression of multidrug efflux pump in Enterobacter aerogenes. Biochem. Biophys. Res. Commun. 328:1113-1118.
23. Gonny, M., P. Bradesi, and J. Casanova. 2004. Identification of the components of the essential oil from wild Corsican Daucus carota L. using 13C-NMR spectroscopy. Flavour Fragr. J. 19:424-433.
24. Hammer, K. A., C. F. Carson, and T. V. Riley. 1999. Antimicrobial activity of essential oils and other plant extracts. J. Applied Microbiol. 86:985-990.
25. Holloway, B. W., and A. F. Morgan. 1986. Genome organization in Pseudomonas. Annu. Rev. Microbiol. 40:79-105
26. **I** 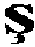 **can, G.,** N. Kirimer, M. Kürkcüoĝlu, K. Hüsnü Can Baser, and F. Demirci. 2002. Antimicrobial screening of Mentha piperita essential oils. J. Agric. Food Chem. 50:3943-3946.
27. Jennings, W., and T. Shibamoto. 1980. Qualitative analysis of flavour and fragrance volatiles by glass-capillary gas chromatography. New York: Academic Press.
28. Köning, W. A., D. H. Hochmunth, and D. Joulain. 2001. Library called MassFinder 2.1 of Terpenoids and Related Constituents of Essential Oils. Hamburg:Scientific Consulting.
29. Li, X. Z., and H. Nikaido. 2004. Efflux-mediated drug resistance in bacteria. Drugs. 64(2):159-204.
30. Lis-Balchin, M., and S. G. Deans. 1996. Antimicrobial effects of hydrophilic extracts of Pelargonium species (Geraniaceae). Lett. Appl. Microbiol. 23(4):205-207.
31. Lomovskaya, O., M. S. Warren, A. Lee, J. Galazzo, R. Fronko, M. Lee, J. Blais, D. Cho, S. Chamberland, T. Renau, R. Leger, S. Hecker, W. Watkins, K. Hoshino, H. Ishida, and V. J. Lee. 2001. Identification and characterization of inhibitors of multidrug resistance efflux pumps in Pseudomonas aeruginosa: novel agents for combination therapy. Antimicrob. Agents Chemother. 45:105-116.
32. Lomovskaya, O., and K. A. Bostian. 2006. Practical applications and feasibility of efflux pump inhibitors in the clinic: a vision for applied use. Biochem. Pharmacol. 71:910-918.
33. Lota, M. L., D. de Rocca Serra, C. Jacquemond, F. Tomi, and J. Casanova. 2001. Chemical variability of peel and leaf essential oil of sour orange. Flavour Fragr. J. 16:89-96.
34. Mahamoud, A., J. Chevalier, A. Davin-Regli, J. Barbe, and J. M. Pagès. 2006. Quinoline derivatives as promising inhibitors of antibiotic efflux pump in multidrug resistant Enterobacter aerogenes isolates. Curr. Drug Targets. 7:843-847.
35. Mahamoud, A., J. Chevalier, S. Alibert-Franco, W. V. Kern, and J. M. Pagès. Antibiotic efflux pumps in Gram-negative bacteria: the inhibitor response strategy. J Antimicrob. Chemother., in press.
36. Malléa, M., J. Chevalier, C. Bornet, A. Eyraud, A. Davin-Regli, C. Bollet, and J. M. Pagès. 1998. Porin alteration and active efflux: two in vivo drug resistance strategies used by Enterobacter aerogenes. Microbiology 144:3003-3009.
37. Mariotti, J. P., F. Tomi, J. Casanova, J. Costa, and F. Bernardini. 1997. Composition of the essential oil of Cistus ladaniferus L. cultivated in Corsica (France). Flavour and Fragr. J. 12:147-151.
38. Martins, M., D. Ordway, M. Kristiansen, M. Viveiros, C. Leandro, J. Molnar, and L. Amaral. 2005. Inhibition of the Carpobrotus edulis methanol extract on the growth of phagocytosed multidrug-resistant Mycobacterium tuberculosis and methicillin-resistant Staphylococcus aureus. Fitoterapia 76:96-9.
39. McLafferty, F. W., and D. B. Stauffer. 1994. Wiley Registry of Mass Spectra Data, 6th Ed. Mass spectrometry Library Search System Bench-Top/PBM, Version 3.10d. Newfield: Palisade.
40. Molnar, P., M. Kawase, K. Satoh, Y. Sohara, T. Tanaka, S. Tani, H. Sakagami, H. Nakashima, N. Motohashi, N. Gyemant, and J. Molnar. 2005. Biological activity of carotenoids in red paprika, Valencia orange and Golden delicious apple. Phytother. Res. 19:700-707.
41. Musumeci, R., A. Speciale, R. Costanzo, A. Annino, S. Ragusa, A. Rapisarda, M. S. Pappalardo, and L. Iauk. 2003. Berberis aetnensis C. Presl. extracts: antimicrobial properties and interaction with ciprofloxacin. Int. J. Antimicrob. Agents. 22:48-53.
42. National Institute of Standards and Technology. 1999. PC version 1.7 of the NIST/EPA/NIH Mass Spectral Library. Norwalk: Perkin Elmer Corp.
43. Ohno, T., K. Masakazu, Y. Yamaoka, S. Imamura, T. Yamamoto, S. Mitsufuji, T. Kodama, K. Kashima, and J. Imanishi. 2003. Antimicrobial activity of essential oils against Helicobacter pylori. Helicobacter 8:207-215.
44. Okusu, H., D. Ma, and H. Nikaido. 1996. AcrAB efflux pump plays a major role in the antibiotic resistance phenotype of Escherichia coli multiple-antibiotic-resistance (Mar) mutants. J. Bacteriol. 178:306-308.
45. Ordway, D., J. Hohmann, M. Viveiros, A. Viveiros, J. Molnar, C. Leandro, M. J. Arroz, M. A. Gracio, and L. Amaral. 2003. Carpobrotus edulis methanol extract inhibits the MDR efflux pumps, enhances killing of phagocytosed S. atreus and promotes immune modulation. Phytother. Res. 17(5):512-519.
46. Pagès, J. M., M. Masi, and J. Barbe. 2005. Inhibitors of efflux pumps in gram-negative bacteria. Trends Mol. Med. 11(8):382-389.
47. Payne, D. J., M. N. Gwynn, and D. L. Pompliano. 2007. Drugs for bad bugs: confrontating the challenges of antibacterial discovery. Nat. Rev. Drug Discov. 6:29-40.
48. Peñalver, P., B. Huerta, C. Borge, R. Astorga, R. Romero, and A. Perea. 2005. Antimicrobial activity of the five essential oils against origin strains of the Enterobacteriaceae family. APMIS 113:1-6.
49. Pradel, E., and J. M. Pagès. 2002. The AcrAB-TolC efflux pump contributes to multidrug resistance in the nosocomial pathogen Enterobacter aerogenes. Antimicrob. Agents Chemother. 46:2640-2643.
50. Rezzi, S., A. Bighelli, D. Mouillot, and J. Casanova. 2001. Composition and chemical variability of the needle essential oil of Pinus nigra subsp. laricio from Corsica. Flavour Fragr. J. 16:379-383.
51. Rossi, P. G., J. Panighi, A. Luciani, D. de Rocca Serra, J. Maury, M. Gonny, A. Muselli, J. M. Bolla, and L. Berti. Antibacterial action of essential oils from Corsica. J. Essential Oil Res., in press.
52. Schelz, Z., J. Molnar, and J. Hohmann. 2006. Antimicrobial and antiplasmid activities of essential oils. Fitoterapia 77(4):279-85.
53. Smith, E. C. J., E. M. Williamson, N. Wareham, G. W. Kaatz, and S. Gibbons. 2007. Antibacterials and modulators of bacterial resistance from the immature cones of Chamaecyparis lawsoniana. Phytochem. 68:210-217.
54. Stavri, M., L. J. Piddock, and S. Gibbons. Bacterial efflux pump inhibitors from natural sources. J. Antimicrob. Chemother., in press.
55. Stezer, W. N., B. Vogler, J. M. Schmidt, J. G. Leahy, and R. Rives. 2004. Antimicrobial activity of Artemisia douglasiana leaf essential oil. Fitoterapia 75:192-200.
56. Viveiros, M., I. Portugal, R. Bettencourt, T. C. Victor, A. M. Jordaan, C. Leandro, D. Ordway, and L. Amaral. 2002. Isoniazid-induced transient high-level resistance in Mycobacterium tuberculosis. Antimicrob. Agents Chemother. 46:2804-2810.
57. Viveiros, M., A. Jesus, M. Brito, C. Leandro, M. Martins, D. Ordway, A. M. Molnar, J. Molnar, and L. Amaral. 2005. Inducement and reversal oftetracycline resistance in Escherichia coli K-12 and expression of proton gradient-dependent multidrug efflux pump genes. Antimicrob. Agents Chemother. 49:3578-3582.

## Claims

1. Geraniol or any of its saturated or unsaturated monoterpene derivatives for use in reducing antibiotic or antiseptic resistance or restoring antibiotic or antiseptic sensitivity to an antibiotic or antiseptic -resistant bacterial strain.

2. Geraniol or any of its derivatives according to claim 1, for use in treating a subject infected with an antibiotic-resistant bacterial strain.

3. Geraniol or any of its derivatives according to claim 1, for use in treating a subject infected with an antiseptic-resistant bacterial strain.

4. Geraniol or any of its derivatives according to claim 1, for use in disinfecting a product infected with an antibiotic-resistant bacterial strain.

5. Geraniol or any of its derivatives according to claim 1, for use in disinfecting a product infected with an antiseptic-resistant bacterial strain.

6. Geraniol or any of its derivatives according to any of claims 1 to 5, wherein the bacterial strain is a Gram-negative bacterial strain.

7. Geraniol or any of its derivatives according to any of claims 1 to 6, for use in combination with an antibiotic or an antiseptic.

8. Geraniol or any of its derivatives according to claim 1, wherein the antibiotic is selected from the group consisting of beta-lactam antibiotics, macrolides, monobactams, rifamycins, tetracyclines, chloramphenicol, clindamycin, lincomycin, fusidic acid, novobiocin, fosfomycin, fusidate sodium, capreomycin, colistimethate, gramicidin, minocycline, doxycycline, bacitracin, quinolones, fluoroquinolones, vancomycin, and trimethoprim.

9. Geraniol or any of its derivatives according to claim 1, for use in a patient infected with enteropathogenic or uropathogenic bacterial strains.

10. Geraniol or any of its derivatives according to claim 1, for use in a patient infected with a bacterium selected from the group consisting of *Enterobacter aerogenes, Escherichia coli, Pseudomonas aeruginosa, Acinetobacter baumannii, Klebsellia, Salmonella, Campylobacter* and *Helicobacter* species.

11. Geraniol or any its derivatives according to any of claims 1 to 10, wherein the derivative is selected from the group consisting of nerol, geranyl acetate, neryl acetate, geranial, neral, citronellol, linalool, tetrahydrogeraniol, myrcenol, and geranyl acetol.

12. Geraniol or any of its derivatives according to any of claims 1 to 11, as inhibitors of bacterial efflux pumps.
